# EUROPEAN PATENT SPECIFICATION

(11) **EP 3 927 823 B1**
(45) Date of publication and mention of the grant of the patent: **24.09.2025**
(21) Application number: 20759691.7
(22) Date of filing: 22.02.2020
(51) Int. Cl.: C12Q 1/6804

(54) **A NOVEL IMMUNO-PCR METHOD USING CDNA DISPLAY**
NEUES IMMUNO-PCR-VERFAHREN MIT CDNA-DISPLAY
NOUVEAU PROCÉDÉ D'IMMUNO-PCR UTILISANT UN AFFICHAGE D'ADNC

(30) Priority: 22.02.2019 JP 2019030509
(43) Date of publication of application: 29.12.2021
(73) Proprietor: Epsilon Molecular Engineering, Inc., Saitama City, Saitama 338-8570 (JP); Saitama University, Saitama-shi Saitama 338-8570 (JP)
(72) Inventor: NEMOTO Naoto, Saitama-shi, Saitama 338-8570 (JP); ANZAI Hironori, Saitama-shi, Saitama 338-8570 (JP); SUZUKI Takeru, Saitama-shi, Saitama 338-8570 (JP); KUMACHI Shigefumi, Saitama-shi, Saitama 338-0824 (JP); TERAI Takuya, Saitama-shi, Saitama 338-8570 (JP)
(74) Representative: Kuhnen & Wacker Patent- und Rechtsanwaltsbüro PartG mbB
(86) International application number: PCT/JP2020/007271
(87) International publication number: WO 2020/171236

(56) References cited:
- LE CHANG ET AL: "Immuno-PCR: An ultrasensitive immunoassay for biomolecular detection", ANALYTICA CHIMICA ACTA, vol. 910, 8 January 2016 (2016-01-08), AMSTERDAM, NL, pages 12 - 24, XP055662017, ISSN: 0003-2670, DOI: 10.1016/j.aca.2015.12.039
- RYAZANTSEV D Y ET AL: "Immuno-PCR: achievements and perspectives", BIOCHEMISTRY (MOSCOW), PLEIADES PUBLISHING, MOSCOW, vol. 81, no. 13, 6 January 2017 (2017-01-06), pages 1754 - 1770, XP036126702, ISSN: 0006-2979, [retrieved on 20170106], DOI: 10.1134/S0006297916130113
- UENO SHINGO ET AL: "cDNA Display: Rapid Stabilization of mRNA Display", ANTIBODY-DRUG CONJUGATES; IN: METHODS IN MOLECULAR BIOLOGY; ISSN 1064-3745; VOL. 263; [METHODS IN MOLECULAR BIOLOGY; ISSN 1064-3745], HUMANA PRESS, US, vol. 805, 1 January 2012 (2012-01-01), pages 113 - 135, XP009190355, ISBN: 978-1-62703-541-5, DOI: 10.1007/978-1-61779-379-0_8
- HIROKI ANZAI, TERAI TAKUYA, JAYATHILAKE CHATHUNI, SUZUKI TAKERU, NEMOTO NAOTO: "A novel immuno-PCR method using cDNA display", ANALYTICAL BIOCHEMISTRY, vol. 578, 1 August 2019 (2019-08-01), pages 1 - 6, XP055729171, ISSN: 0003-2697, DOI: 10.1016/j.ab.2019.04.017
- SHARIF HASAN, JINHUA DONG, YUKO HARA, YOSHIHITO MORIZANE, FUTOSHI SHIBASAKI, HIROSHI UEDA: "Protein-based Open Sandwich Immuno-PCR for Sensitive Detection of Small Biomarkers", ANALYTICAL SCIENCES, 30 September 2013 (2013-09-30), pages 871 - 876, XP055729173, ISSN: 0910-6340, DOI: 10.2116/analsci.29.871
- TAKERU SUZUKI, MOCHIZUKI YUKI, KIMURA SHINNOSUKE, AKAZAWA-OGAWA YOKO, HAGIHARA YOSHIHISA, NEMOTO NAOTO: "Anti-survivin single-domain antibodies derived from an artificial library including three synthetic random regions by in vitro selection using cDNA display", BIOCHEMICAL AND BIOPHYSICAL RESEARCH COMMUNICATIONS, vol. 503, no. 3, 10 September 2019 (2019-09-10), pages 2054 - 2060, XP055729175, ISSN: 0006-291X, DOI: 10.1016/j.bbrc.2018.07.158
- MOCHIZUKI YUKI; SUZUKI TAKERU; FUJIMOTO KENZO; NEMOTO NAOTO: "A versatile puromycin-linker using cnvK for high-throughputin vitroselection by cDNA display", JOURNAL OF BIOTECHNOLOGY, vol. 212, 28 August 2015 (2015-08-28), pages 174 - 180, XP029284068, ISSN: 0168-1656, DOI: 10.1016/j.jbiotec.2015.08.020

## Description

### Technical Field

The Present invention relates to a novel immuno-PCR method. Specifically, it relates to the novel immuno-PCR method by using cDNA display.

### Background Art

Detection of biomarkers that exist in biological samples (for example, serum, urine, etc.) at low concentration is of critical importance in basic biology and in diagnosis. Immuno-PCR (it is referred to as "IPCR"), first developed by Sano and others [see non patent document 1, Prior art 1], is a sensitive and quantitative analysis technique for this purpose [see non patent document 2].

The general IPCR approach involves initial capture of a target antigen with a capture antibody coated on a surface and subsequent detection with a detection antibody attached with a DNA reporter. This DNA is amplified by PCR with appropriate primers and fluorescently quantified using real-time PCR apparatus. Because the method combines advantages of immunoassay and PCR, it has both high versatility and exponential signal amplification. In recent years, IPCR has been applied for detection of various antigens including cancer biomarkers and viruses.

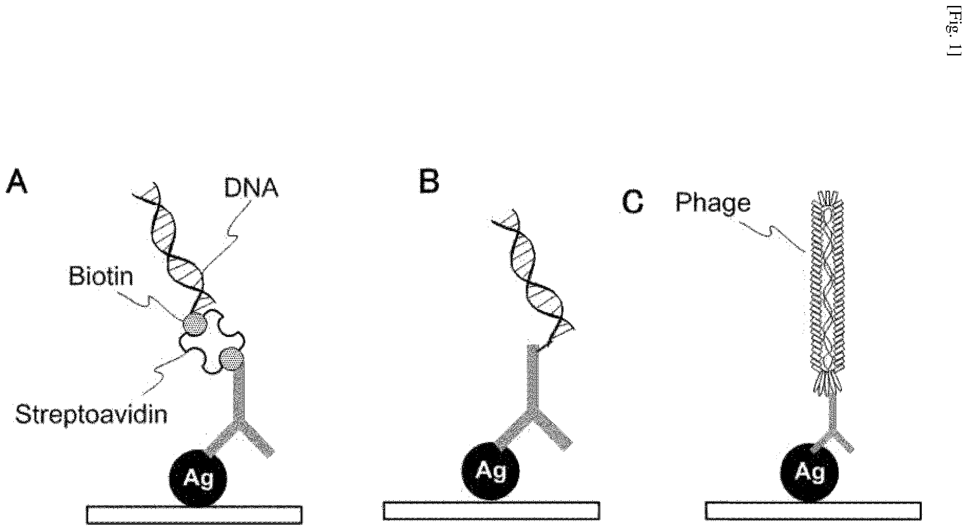

In the development and application of IPCR, how the antibody is conjugated with reporter DNA is a critical issue. Traditionally, streptavidin has been used as a linker for combining biotinylated DNA and biotinylated antibody (Fig. 1A). In Fig. 1A, streptavidin is used to connect biotin-labeled antibody with biotin-labeled DNA reporter. However, the tetrameric nature of streptavidin leads to the formation of heterogeneous DNA-antibody conjugates, which may decrease the reproducibility of IPCR.

Another method relies on chemical cross linkers for directly conjugating DNA to the antibody (Fig. 1B). In Fig. 1B, covalently crosslinked antibody-DNA conjugate is used. Although this can reduce complexity and is straightforward, conventional crosslinking chemistry reacts with all cysteine/lysine residues in the antibody, and the modification may compromise binding affinity of the antibody. Moreover, the number of DNA molecules bound to one antibody is heterogeneous and hard to control. To address these problems, Zhang and others developed an innovative technology: phage display mediated immuno-PCR (PD-IPCR, Fig. 1C). In Fig. 1C, different peptides, including single-chain variable fragment of antibodies and VHH, may be displayed on the surface of M13 or T7 phage. Phage DNA is used as DNA marker.

Phage display was first described by Smith in 1985 [see non patent document 3], and is widely used for directed evolution of polypeptides including antibodies. In PD-IPCR, an engineered bacteriophage M13 (or T7) that expresses a single-chain antibody for the analyte on the surface of the virus is used as a supramolecular complex of an antibody and DNA. After incubation of the phage with the immobilized antigen, bacterial DNA is amplified for quantification. The use of phage particles in IPCR obviates the tedious preparation of an antibody-DNA conjugate, and provides low cost and highly sensitive assays for proteins, toxins, and viruses [see patent document 4 & 5].

### Prior Art

### Non-Patent document

Non-Patent Document 1: T. Sano, et al., Science, 258 (1992) 120-122.
Non-Patent Document 2: L. Chang, et al., Anal. Chim. Acta., 910 (2016) 12-24.
Non-Patent Document 3: G. P. Smith, Filamentous fusion phage: novel expression vectors that display cloned antigens on the virion surface, Science, 228 (1985) 1315-1317.
Non-Patent Document 4: R. Monjezi, et al., J. Virol. Methods, 187 (2013) 121-126.
Non-Patent Document 5: J. Lei, et al., Anal. Chem., 86 (2014) 10841-10846.
Hasan et al. (2013), Anal. Sci. 29: 871-876 , relates to a sandwich immuno-PCR method discussing the disadvantages of phage display.

### Summary of Invention

### Problem to be solved by the invention

Nevertheless, PD-IPCR has still some problems. Because strict control on the number of exposed engineered coat protein is difficult, heterogeneity of DNA-protein linkage is not completely solved. Further, because these bacteriophages are genetically engineered virus, experiments must be performed under strict legal regulation, which may be a hurdle for some real-world applications. Hence, it is important to build a novel IPCR system that is 1) based on strictly homogeneous DNA-antibody conjugation, 2) non-viral, and 3) easy to perform by biologists without knowledge of organic synthesis.

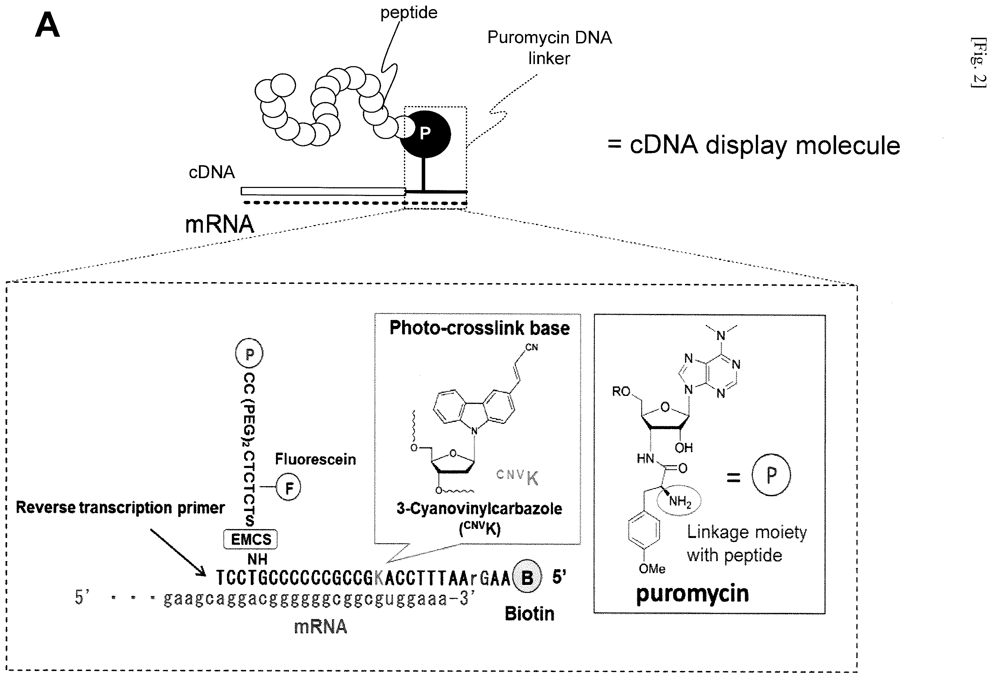

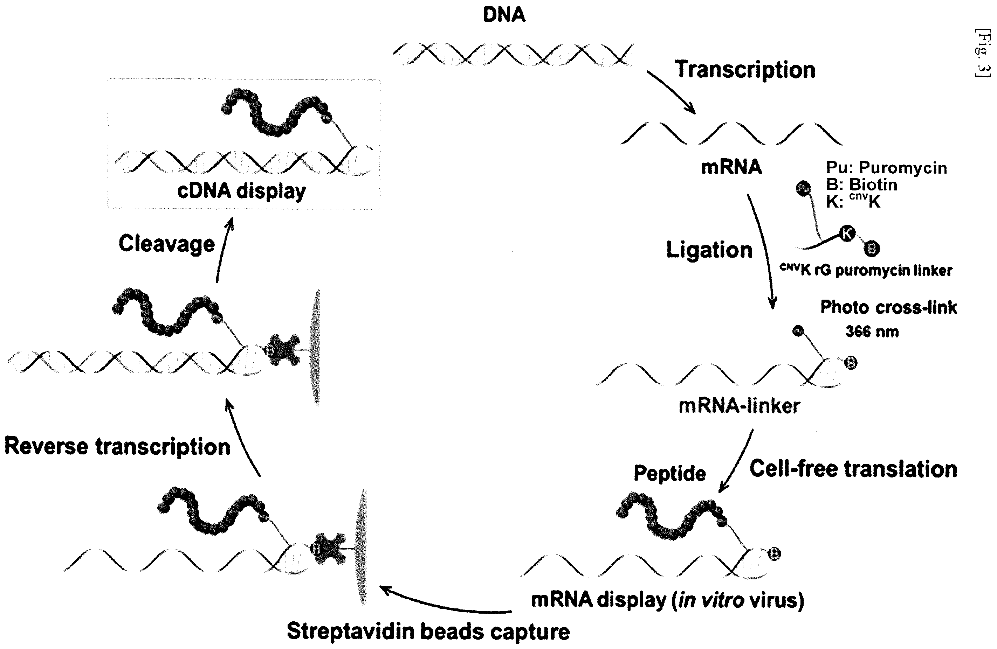

To address this issue, after thorough investigation, we invented a novel IPCR method using "cDNA display" as a system that fulfills the above requirements. The cDNA display molecule is a single molecule conjugate of cDNA and its coded polypeptide (Fig. 2A), which is biochemically synthesized from mRNA by successive ligation with a puromycin DNA linker, in vitro translation, and reverse-transcription (Fig. 3). Compared with phage display, cDNA display has advantages in terms of library size (10¹³/mL) and it is stable under harsh conditions such as organic solvents, strong acid/base, and heat. Further, because of the conjugation mechanism, DNA-peptide ratio is always 1:1. So far, we and others have applied cDNA display for in vitro selection of peptides that had affinity to proteins, RNAs, and lipid membranes, and small molecule.

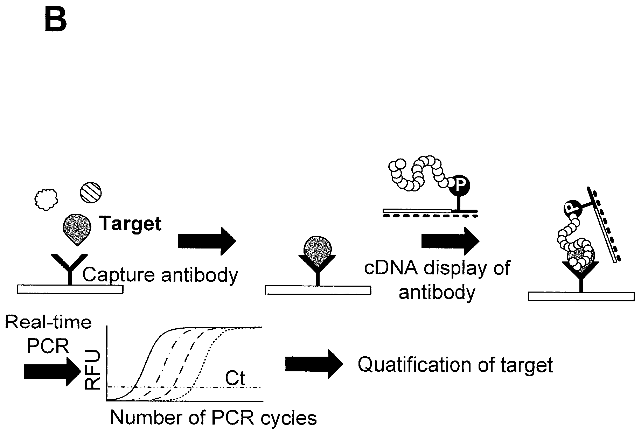

Stimulated by PD-IPCR, we envisioned that cDNA display molecules can also be regarded as antibody-DNA conjugates used for IPCR, and implemented the idea as described below. The newly developed method, which we termed cDNA display mediated immuno-PCR (cD-IPCR, Figs. 2B), was successfully applied to the detection of a model target protein (GFP) spiked in serum. Although a few other protein-DNA conjugation systems at the single-molecule level have also been reported, such as ribosome display and mRNA display, these complexes are not stable under physiological conditions such as in serum.
Hence, we believe cD-IPCR takes an advantage of the stability of cDNA display.

### Means for Solving the problem

Under the above-mentioned circumstances, the inventors of the present invention completed the present invention. Namely, one aspect of the present invention is an improved immuno-PCR method by using cDNA display comprising the steps of: immobilizing a first antibody having a binding site to a solid phase: contacting a sample fluid to said antibody to bind a target molecule in said sample fluid; contacting said target molecule to a cDNA display being composed of 1) a backbone nucleic acid strand in which a DNA sequence to be amplified is included and 2) a covalently-bound side chain strand having a polypeptide conjugation site to which the second antibody that binds to the target molecule has been conjugated; and conducting polymerase chain reaction to detect said cDNA quantitatively, wherein said polypeptide conjugation site is composed of any one of compound selected from the group consisting of puromycin and derivative thereof, said backbone nucleic acid strand comprises a double strand of mRNA and cDNA, said backbone strand comprises the cDNA sequence of the second antibody which binds to the target molecule, and the second antibody is a single domain antibody, that binds to a different epitope of the target molecule from the first antibody.

Here, the first antibody is bound to the solid phase without anything or with something, for example, a small molecule, a protein, a peptide, or artificial polymers. It is preferably bound to the solid phase via biotin-streptavidin interaction.

Said first antibody is anyone selected from the group consisting of IgG, a single domain antibody, and a fragment thereof that binds to epitope of the target molecule contained in the target molecule.

Said PCR is preferably quantitative PCR.

### Advantageous Effects of the Invention

According to the improved immuno-PCR method of the present invention, the target molecule in the sample fluid is detected and measured quantitatively, because it uses cDNA display being composed of one protein/peptide and one DNA. Also, according to the method of the present invention, the suitable second antibody against the given target molecule may be selected by using cDNA display technique; and then the identified second antibody is utilized for cD-IPCR without laborious optimization.

### Brief Description of Drawings

Fig. 1 shows schematic representation of cDNA display mediated immune-PCR (cD-IPCR). Fig 1 (A) to (C) show previously developed immuno-PCR formats. Fig. 1(A) is an original universal system of immuno-PCR using streptavidin. Fig. 1(B) shows a chemically crosslinked antibody-DNA conjugate. Fig. 1(C) shows phage display mediated immuno-PCR (PD-IPCR). "Ag" shows an antigen.
Fig. 2 is a schematic representation of cDNA display, detection method, sensitivity, and detection using cDNA display. Fig. 2(A) shows the schematic structure of cDNA display, which is a covalent peptide-DNA complex conjugated via a puromycin linker. The puromycin linker makes an amide bond with the nascent peptide at its C-terminus in the process of ribosomal translation reaction. In this study, mRNA is hybridized to cDNA using a photo-crosslinking base (3-cyanovinylcarbazole, which is referred to as "cnvK" hereinbelow). Expanded figure shows the detailed chemical structure of the puromycin linker moiety.
Fig. 2 (B) is a schematic diagram of cD-IPCR (sandwich-type detection). A target protein in a biological sample is captured on solid phase (beads or plates) using a capture (or the first) antibody. After washing, cDNA display of a polypeptide that has affinity to the target is added. Unreacted display molecules are washed, and resulting cDNA is quantified by qPCR. Here, cDNA display can be used both as an antibody for the target and also as a DNA marker for PCR quantification, which is the main point of this study.
Fig. 3 schematically shows preparation of cDNA display. Detailed protocol of each step is described in the example.

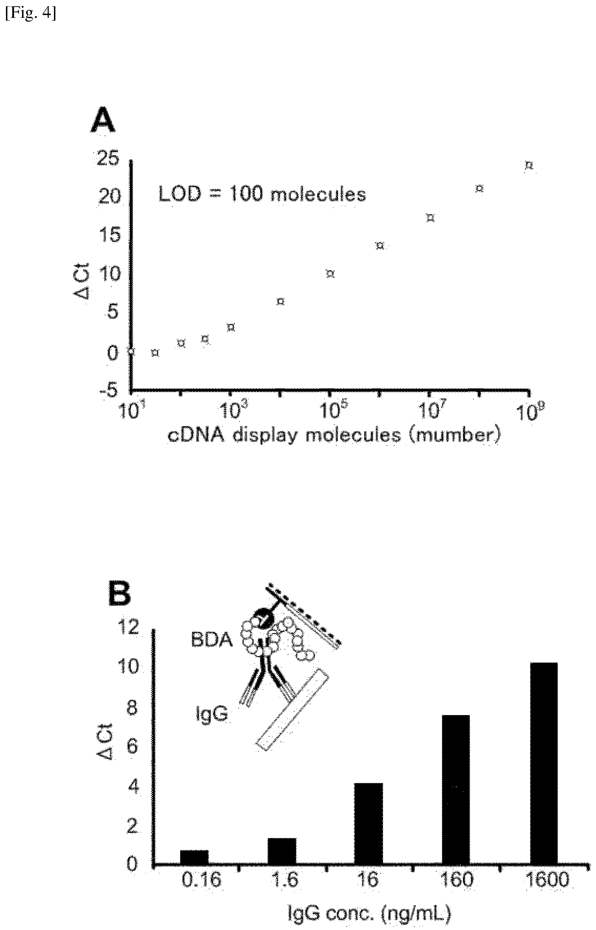

Fig. 4 shows graphs for sensitivity and linearity of qPCR of cDNA display molecules (Fig. 4A), and the result of IgG detection with cD-IPCR (Fig 4B). In Fig. 4A, cDNA display molecules presenting BDA (a peptide that binds to IgG) were serially diluted in PCR tubes, and qPCR was performed with an appropriate primer set. Difference of threshold cycles (ΔCt) between the sample and the control (no template) was calculated. Data are shown as mean ± S.D. (n=3). In Fig. 4B, IgG immobilized on solid phase was serially diluted and reacted with cDNA display presenting BDA. qPCR was performed with an appropriate primer set. ΔCt was calculated as the difference between the sample and the control (without target).

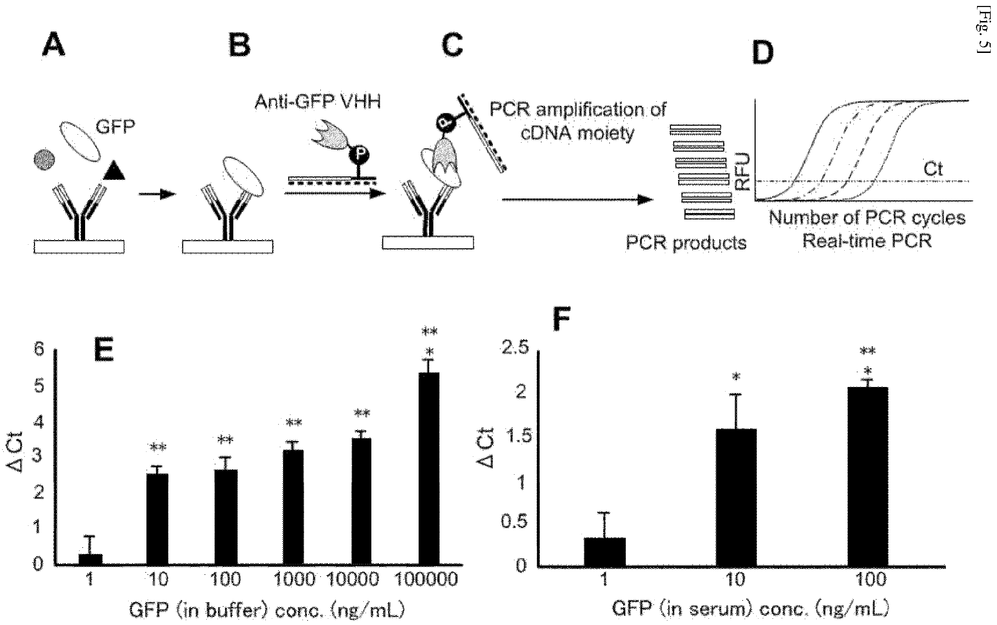

Fig. 5 shows a schematic diagram of cD-IPCR in GFP-VHH model, and detection of GFP with cD-IPCR method. All of Fig. 5(A) to (D) show the schematic diagram of cD-IPCR. Anti-GFP IgG was used as the first antibody, and cDNA display presenting anti-GFP VHH was used as detection agent. Fig. 5(E) is the graph showing the detection of GFP in buffer, and Fig 5(F) shows that spiked in serum. ΔCt was calculated as the difference between the sample and the control (without target).

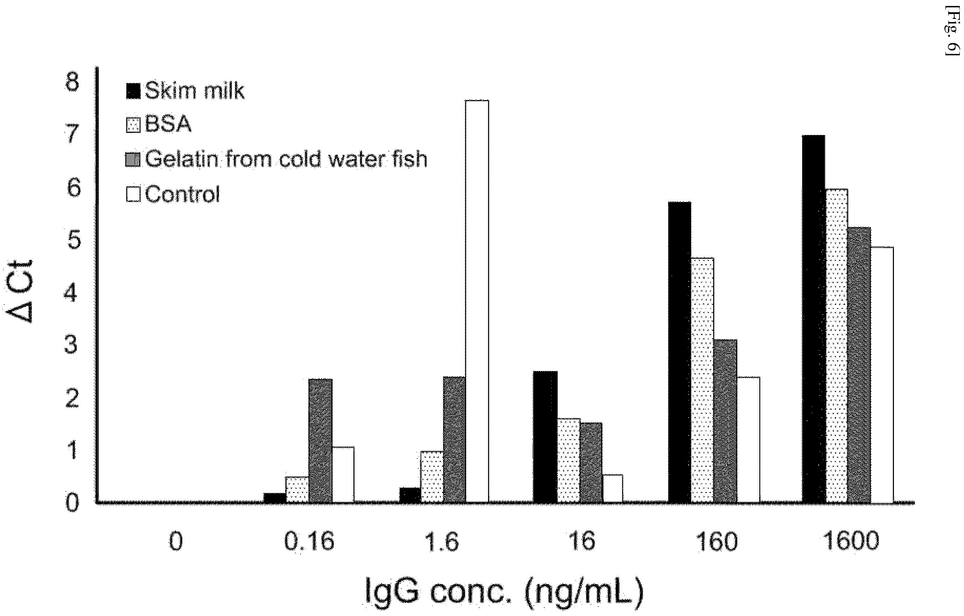

Fig. 6 shows comparison of blocking agents. Each blocking agent was used for IgG-immobilized magnetic beads (Magnosphere MS300/Streptavidin), and cDNA display of BDA was incubated with the beads. Collected DNA was evaluated by qPCR. ΔCt was calculated as the difference between the sample and the control without target. Quantitative amplification was observed with skim milk (SM) and BSA. With gelatin (from cold water fish) and control (w/o any blocking agent), obtained data were not concentration-dependent.

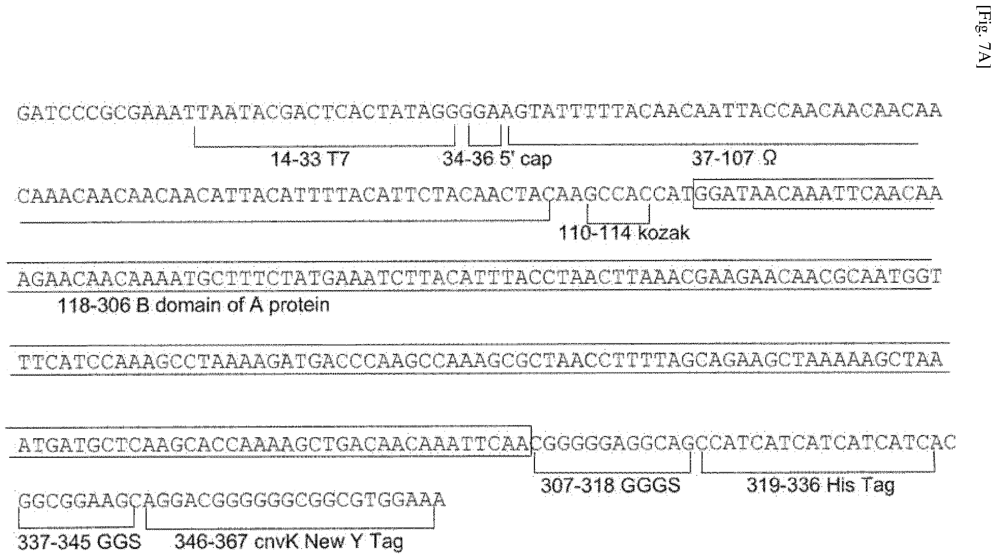

Fig. 7A is BDA full-length construct.

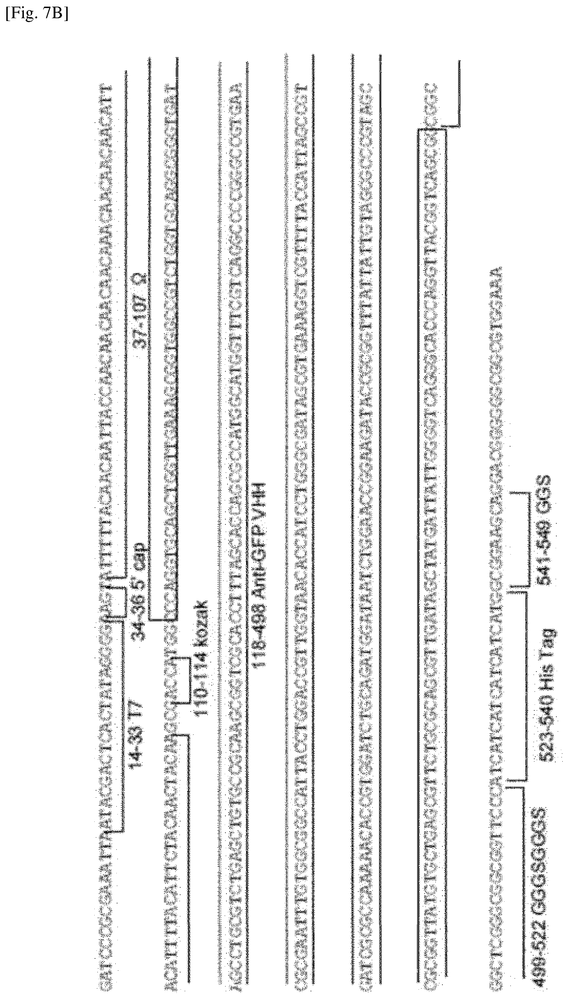

Fig. 7B is Anti-GFP VHH full-length construct.

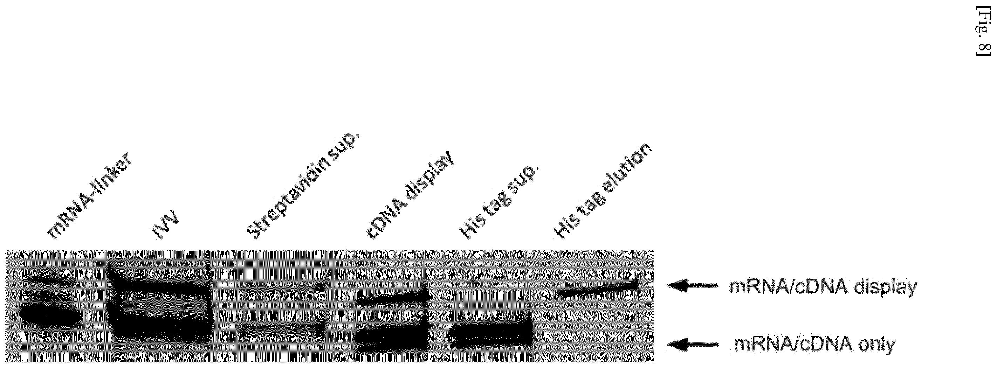

Fig. 8 is confirmation of cDNA display formation of BDA by using gel electrophoresis.

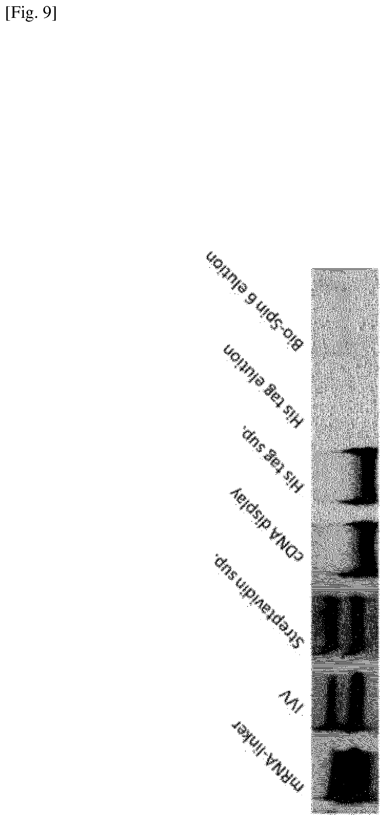

Fig. 9 is confirmation of the anti-GFP VHH coding cDNA display formation by using gel electrophoresis.

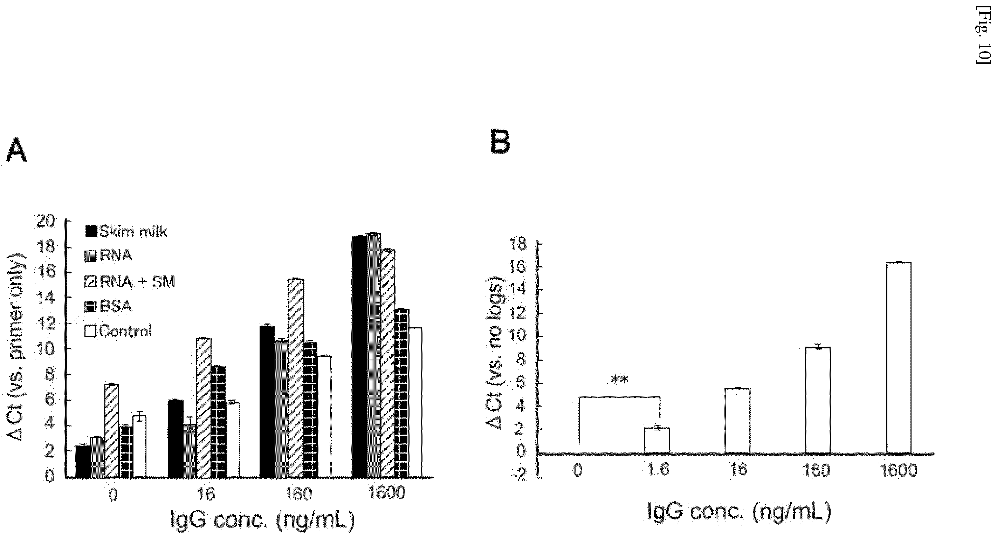

Fig. 10 is a proof-of-concept experiment of direct cD-IPCR. cDNA display of BDA was incubated with different amounts of its target protein (IgG) that was immobilized on streptavidin beads (Dynabeads MyOne streptavidin C1 streptavidin). qPCR was performed and Ct values were determined. Fig. 10A: Comparison of blocking agents (from left to right: skim milk, tRNA from yeast, mixture of skim milk and tRNA, BSA, and no blocking agent). ΔCt against no template (primer only) control was calculated, and data are shown as mean ± S.D. (n = 3) Fig. 10B: Quantification of IgG in direct cD-IPCR format. Skim milk (5%, w/v) was used as blocking agent. ΔCt against no IgG control was calculated, and data are shown as mean ± S.D. (n = 3). ** indicates p < 0.01 (versus 0 ng/mL, t-test). For every other pair of columns, statistical significance (p < 0.001) was confirmed. For detailed protocols, see examples.

### Embodiments

Herein below, the present invention is explained in detail by using drawings.

### 1. Construction of DNA

Firstly, we prepared the DNA construct which comprises coding sequence of B domain of A protein (BDA whole, Fig. 7A). The BDA whole is comprised of the sequences coding BDA (BDA gene), T7 promoter, translation promoting sequence (W), spacer region, Histidine tag (His tag), and a sequence having complementary strand to a part of the puromycin linker. Among them, DNA fragment being composed of T7 promoter sequence and translation promoting sequence is added to 5' upstream of BDA gene. Histidine tag (His tag) and a sequence having complementary strand to a part of the puromycin linker is added to 3' downstream of BDA gene.

The BDA whole is synthesized by a standard molecular biology technique using PCR, and then it was purified by using the conventional method. Then, they are transcribed to RNA by using T7 RiboMAX Express Large Scale RNA Production System (Promega, Inc.) according to a protocol attached to the kit, to obtain about 5 to 30 pmol/ µL of mRNA (SEQ ID No. 1).

Anti-GFP VHH construct (Fig. 7B) is prepared as follows: First, a gene coding anti-GFP VHH (LaG-16 in P.C. Fridy, et al., Nat. Methods 11 (2014) 1253 - 1260.), which is chemically synthesized by GenScript, was amplified by PCR (25 cycles, 50 µL scale) with 1st Fw VHH and 1st Rv VHH as primers (for primer sequences, SEQ ID Nos. 8 & 9, see Table 2). Second PCR to attach 5'-UTR is then performed (25 cycles, 50 µL scale) with the DNA described above as the template and T7Ω and cnvK-NewYtag as primers (SEQ ID Nos. 7 & 6, see Table 2). The product is purified by preparative PAGE (4%, denaturing conditions).

The synthesis of puromycin linker used in this invention (sometimes, it is referred to as "cnvK linker") is prepared as described in below. The modified oligonucleotides Puro-F-S2 and the biotin-rG-cnvK fragment may be ordered to a DNA synthesis company (Tsukuba Oligo Service, for example). The Puro-F-S2 fragment represents 5'-(S)-TC-(F)-CTC-(Spacer 18)-(Spacer 18)-CC-(Puro)-3', where S is the 5'-thiol Modifier C6, F is fluorescein-dT, Puro is puromycin CPG and Spacer 18 is the spacer phosphoramidite 18. The biotin-rG-cnvK fragment and it represents 5'-(B)-AA-(rG)-AATTTCCA-(K)-GCCGCCCCCCG-(T)-CCT - 3', where B is 5'-biotinTEG, K is cnvK, and T is the amino-modifier C6 dT. The cross-linking reaction of the Puro-F-S2 fragment and the biotin-rG-cnvK fragment via N-(6-maleimidecaproyloxy) succinimide (EMCS, Dojindo laboratories, Kumamoto, Japan) is performed according to a method described in the previous report (Yamaguchi et al, Nucleic Acids Res. 37 (2009) e108; Mochizuki et al ACS Comb. Sci. 13 (2011) 478-485).

A predetermined amount of the Puro-F-S2 fragment is reduced by predetermined concentration of dithiothreitol in the predetermined concentration of disodium hydrogen phosphate for the predetermined time period and temperature and then desalted on a NAP-5 column (GE Health care, Waukeshu, WI USA) just before use. For example, 30 nmol of the Puro-F-S2 fragment is reduced by 50 mM dithiothreitol in 1 M disodium hydrogen phosphate for 1 hr at room temperature. Then, the reduced fragment is desalted, for example, by using NAP-5 column (GE Health care, Waukeshu, WI USA) just before use.

Predetermined volume of biotin-rG-cnvK fragment and EMCS are mixed in the predetermined volume of sodium phosphate buffer. For example, a total of about 5 to 15 nmol of the biotin-rG-cnvK fragment and about 1 to 3 mmol of EMCS are mixed in about 50 to 150 µL of about 0.1 to 0.3 M sodium phosphate buffer (pH about 7.0 to 7.5).

The mixture is subsequently incubated for predetermined time and temperature, and excess EMCS is removed by ethanol precipitation. For example, the mixture is subsequently incubated for 30 minutes at 37 °C and excess EMCS was removed by ethanol precipitation with a co-precipitating agent such as Quick-Precip Plus Solution (Edge BioSystems, Gaithersburg, MD USA) and so forth. The reduced Puro-F-S2 fragment is immediately added to the precipitate and incubated at low temperature and predetermined time period, for example, 4 °C overnight.

In order to stop the reaction, dithiothreitol (DTT) is added to the sample at the predetermined concentration, and incubated for predetermined time and temperature. For example, final concentration of DTT is about 40 to 60 mM, and incubation time and temperature are about 20 to 40 minutes at around 35 to 40 °C. In order to remove non-reacted Puro-P-S2 fragment, ethanol precipitation may be conducted by using the co-precipitating agent as described above. The precipitate is dissolved with nuclease free water and purified by using C18 HPLC column under the predetermined conditions. See Table 1.

**(Table 1)**

| Item | |
|---|---|
| Column | 4.6 x 250 mm, for example, AR 300 (Nakarai Tesuque, Kyoto, Japan) |
| Solvent A | Trimethyl Ammonium Acetate, for example, about 0.05 to 0.15 M (TEAA, Glen Research. Sterling, VA USA) |
| Solvent B | acetonitrile/water (for example, 70 to 90: 30 to 10, v/v) |
| Flow rate | about 0.5 to 1.5 ml/min (linear gradient from about 15% to about 35 % B over 45 minutes) |
| Detection | ultraviolet (UV) absorbance at around 250 to 270 nm fluorescence excitation/emission at 485 to 490/510 to 530 nm |

The fractions that form the last peak at UV monitoring, which corresponds to a single fluorescence peak at an emission at around 510 to 530 nm, are collected to obtain cnvK-Pu-linker. After drying the fractions, the cnvK-Pu-linker is resuspended in nuclease free water. For each DNA construct (and its transcribed mRNA), purity and size were checked by denaturing PAGE containing 8 M urea.

### 2. Preparation of cDNA display

The DNA constructs of BDA (SEQ ID NO. 1, Fig. 7A) and anti-GFP VHH (SEQ ID NO. 12, Fig. 7B) are converted to cDNA display according to the conventional method with slight modifications (Fig. 2). Formation of cDNA display was then confirmed by urea SDS-PAGE (Figs. 8 and 9), which is a standard method for such protein-DNA conjugates.

### 3. qPCR quantification of cDNA display in solution

Firstly, it is necessary to confirm whether cDNA display molecules can really be quantified by real-time PCR, which is referred to as "qPCR" herein below, and their sensitivity of detection. The BDA-coding cDNA display molecules are diluted every 10-fold (from about 10³-10⁹copies/about 2 µL) and about 3.3-fold (about 10¹-10³ copies/about 2 µL) by ultrapure distilled water (UPDW). About 2 µL of these solutions are then used as a DNA template for qPCR. For example, qPCR is performed with THUNDERBIRD SYBR qPCR Mix (Toyobo, Japan) using the StepOne Real-Time PCR System (Thermo Fisher Scientific, USA).

The PCR mixture contains, for example, about 1 × THUNDERBIRD SYBR qPCR Mix, about 250 to 350 nM concentration of each primer (forward primer: BDA_qPCR (+), reverse primer: BDA_qPCR (-), (see Table 2, SEQ ID NOs. 3 and 4), about 0.2 to 0.6 µL of about 40 to 60 × ROX reference dye, about 1 to about 3 µL of the cDNA display dilutes and UPDW in a final volume of about 15 to 25 µL. The step program for PCR is as follows: about 94 to about 96 °C for about 0.5 to 1.5 minutes, followed by about 35 to 45 cycles at around 94 to 96 °C for about 10 to 20 second, and around 60 to 65 °C for about 25 to 35 seconds.

The negative control that contains all the qPCR reagents except the DNA template is included to verify the quality of amplification, and the difference of Ct values between the samples and the control is calculated. LOD, namely limit of detection, is determined as the lowest number of detectable template molecules, and analyzed by using t-test.

### 4. Direct cD-IPCR detection of IgG

In direct cD-IPCR, IgG-immobilized magnetic beads (Dynabeads MyOne streptavidin C1, prepared as described in the example) are mixed in 10-fold serial dilution with intact magnetic beads. Final concentrations of IgG in the tubes are set as for example, about 1.6 to about 1600 ng/mL, and 0 ng/mL. Beads are blocked with predetermined blocking agent in an appropriate biding buffer; for example, about 5% (w/v) of skim milk, dispersed in about 1 × SA binding buffer (about 40 to 60 µL, about 8 to 15 mM Tris-HCl, pH around 7.2 to 7.6, including about 0.5 to 1.5 mM EDTA, about 0.5 to 1.5 M NaCl, about 0.05 to about 0.15 % (v/v) Tween 20).

After blocking, the beads are washed for predetermined times with the binding buffer; for example, washed twice with about 50 to 150 µL of around 1 × SA binding buffer. Then, they are reacted with cDNA display coding BDA that is diluted by using about 10 to 30 µL of around 5-fold with about 1×SA binding buffer at around 23 to 27 °C for about 50 to 70 minutes. In the buffer, the amount of cDNA display is about up to 70 to 90 fmol/sample).

After washing the beads, the bound cDNA display is eluted by the appropriate buffer, for example, SDS/DTT buffer of about 50 to about 150 µL, which includes about 0.5 to 1.5 % (v/v) SDS, about 25 to 75 mM DTT, about 25 to 75 mM Tris-HCl, about 0.4 to 0.6 M NaCl, about 0.5 to 1.5 mM EDTA, about 0.04 to 0.06% (v/v) of Tween 20, pH around 7.2 to 7.6, at around 40 to 60 °C for about 25 to 35 minutes. After DNA purification, samples are subjected to qPCR as a template. The qPCR conditions are the same as described above. The difference of Ct values between the samples and the no antigen negative control (0 ng/mL IgG) is calculated.

### 5. Sandwich cD-IPCR detection of GFP in buffer

A polystyrene microtiter plate, for example, MICROLON (Trademark), 96 Well Single-Break Strip Plate, PS, Greiner), is coated overnight at around 4 °C with predetermined antibody such as anti-GFP pAb (MBL, #598) diluted about 1000-fold with PBS, which contains about 5 to 15 mM sodium phosphate buffer with both of about 130 to 145 mM NaCl and about 2.5 to 3.0 mM KCl, pH around 7.2 to 7.6, about 100 µL.

After washing the plate with PBS, it is blocked with the blocking agent, for example, PBS containing about 0.5 to 1.5% (w/v) skim milk by incubation at about 22 to 27 °C for about 1 to 3 hours, and then washed out by using PBS (for example, about 150 to 250 µL, 2 to 4 times). Each 10-fold serial dilution of GFP in PBS from about 100 µg/mL to about 1 ng/mL as well as negative control (no GFP) is applied to the wells at around 22 to 27 °C for about 1 to 3 hours, and then washed out by using PBS (about 150 to 250 µL, 6 to 10 times).

cDNA display coding anti-GFP VHH, is diluted by predetermined fold, for example 60 to 70-fold, with PBS-T as described above. It corresponds to those up to about 1.5 fmol/sample, and is then incubated at about 22 to 27 °C for about 1 to 3 hours. After washing with PBS-T as described above, the bound cDNA display is eluted by using, for example, glycine/HCl buffer (about 0.1 to 0.3 M, pH about 2.0 to 2.4, about 100 µL).

The elute is neutralized with about 0.5 to 1.5 M Tris-base solution, and cDNA display was purified using, for example, a Favorprep kit, for use as the DNA template for qPCR. As qPCR, the same kit as described above may be used and the experiment may be conducted as the same as described above. PCR program may be modified, for example, about 94 to 96 °C for 0.5 to 1.5 minute, followed by about 30 to 50 cycles of about 94 to 96 °C for about 10 to 20 seconds and about 64 to 68 °C for 25 to 35 seconds. The primer only control is also included to verify the quality of amplification. The difference of Ct values between the samples and the negative control without GFP is calculated.

### 6. Sandwich cD-IPCR detection of GFP in serum

The procedures for the sandwich cD-IPCR detection of GFP in serum are similar to that of described above, except that GFP was dissolved in commercially available healthy human serum (Kohjin Bio, Japan) instead of PBS, and used for the reaction.

In this study, qPCR was performed with a system (StepOne, ThermoFisher) using Thunderbird SYBR qPCR mix (Toyobo) reagent and appropriate primers. See following Table 1.

**(Table 2)**

| Table 2. Primers used in this study. | | SEQ ID NO. |
|---|---|---|
| Name | Sequence (5' to 3') | |
| Newleft | GATCCCGCGAAATTAATACGACTCACTATAGGG | 5 |
| cnvK*¹-NewYtag | TTTCCACGCCGCCCCCCGTCCT | 6 |
| T7Ω | GATCCCGCGAAATTAATACGACTCACTATAGGG GAAGTATTTTTACAACAATTACCAACAACAAC AACAAACAACAACAACATTACATTTTACATTC TACAACTACAAGCCACCATG | 7 |
| BDA_qPCR (+) | CTACAAGCCACCATGGATAAC | 3 |
| BDA_qPCR (-) | GCTTGGGTCATCTTTTAGGC | 4 |
| 1st Fw*² VHH | CATTTTACATTCTACAACTACAAGCCACCATGG CCCAGGTGCAGCTG | 8 |
| 1 st Rv*³ VHH | TTTCCACGCCGCCCCCCGTCCTGCTTCCGCCAT GATGATGATGATGATGATGGGAAC | 9 |
| GFPVHHqPCRFw | AACACCATCCTGGGCGATAG | 10 |
| GFPVHHqPCRRv | GTGTTTTTGGCGCGATCAC | 11 |

| | | |
|---|---|---|
| *1: In Table 1, "cnvK" means 3-cyanovinylcarbazole. *2: "Fw" means forward. *3: "Rv" means reverse. | | |

The curves of the amplification data (relative fluorescence units) were analyzed by the attached software, and the Ct values were defined as the number of cycles required for the fluorescent signal to cross the threshold.

As shown in Fig. 4A, ΔCt (difference of Ct values with respect to no template control) was linearly correlated with the number of cDNA display molecules in the range of 10³-10⁹ copies, and the standard deviations were very low. At lower concentrations, whereas linearity was not maintained, statistically significant ΔCt was observed for the sample containing as low as 100 molecules. Both of the low limit of detection (LOD) and a wide detection range (from 10² to 10⁹) are unique advantages of IPCR.

We then moved to the cD-IPCR detection of target protein directly immobilized on solid phase (i.e. direct cD-IPCR). After several conditions such as blocking agents (Fig. 6) and used magnetic beads were optimized, binding of BDA to IgG was used as a model system (Fig. 4B).

Different amount of rabbit IgG was immobilized on magnetic beads, and cDNA display molecules of BDA was incubated with the beads. After washing unbound molecules, BDA-IgG interaction was broken by Gly-HCl treatment and the cDNA in the eluates were quantified by qPCR as above. The results indicated that direct cD-IPCR could detect IgG from 1.6 µg/mL to 160 pg/mL.

Next, we demonstrated that cD-IPCR could be applied to sandwich-type detection scheme using a single-domain antibody (Fig. 5A-D). Single-domain antibodies (also called variable domain of the heavy chain of a heavy chain antibody; VHH, or nanobody) are promising reagents for therapeutics and diagnostics because of their stability, cost-effective production and ease of modification.

Due to its small size (approximately 15 kDa) and single strand nature, VHH is suitable for directed evolution using phage display and cDNA display. Especially, in vitro selection method like this enables us to screen against antigens that cannot be immured by animals for its toxicity. Here, we applied cDNA display encoding anti-GFP VHH for cD-IPCR detection of GFP (P. C. Fridy, et al., Nat. Methods., 11 (2014) 1253-1260.). First, the polyclonal antibodies (pAb) for GFP were immobilized on an ELISA plate by physical adsorption, and varying amount of GFP diluted in PBS was captured on plate.

Then, it was reacted with cDNA display presenting anti-GFP VHH. After acidic elution, DNA was quantified by qPCR. As shown in Fig. 5E, the ΔCt value was gradually increased with the GFP concentration (from 10 to 10⁵ ng/mL), and 10 ng/mL was the lowest detected concentration. Finally, robustness and potential practical utility of the VHH-based cD-IPCR system was evaluated by detection of GFP spiked in human serum (final GFP concentration: 1 to 100 ng/mL). The sensitivity of detection was essentially the same as in the buffer, which indicated that cD-IPCR could be applied for biological samples (Fig. 5F).

In conclusion, we have performed a proof-of-concept study of the novel PCR-based antigen detection method called cD-IPCR. In contrast to ELISA, the signal amplification of cD-IPCR is exponential, resulting in very high sensitivity (~100 molecules/sample) and broad detection dynamic range (> 10⁷fold). Compared with traditional IPCR and PD-IPCR, this method has potential advantages in terms of quantitativity and reproducibility due to intrinsic one-to-one conjugation ratio of cDNA and its coding protein.

We believe that the method developed here has broad applicability and practical utility in immunoassays of a wide variety of antigens. Also, like PD-IPCR, the newly developed method can be coupled with in vitro selection of optimized polypeptides from huge libraries. For this reason, it should be possible to rapidly screen a new binding peptide (or a single-chain antibody) for a given target substance with cDNA display, and use the identified binder for cD-IPCR. Of course, like other IPCR, the sensitivity of the assay highly depends on a number of factors including the affinity of the capture/detection antibodies, extent of non-specific binding of the DNA-antibody conjugates, and choice of primer pairs.
Following examples explain one of the features of the present invention and do not limit to the scope of the present invention.

### Example

### (Example 1)

### (1) Materials and general instruments

General chemicals were of the best grade available, supplied by Tokyo Chemical Industries (Japan) and Wako Pure Chemical Industries (Japan). Chemicals for molecular biology experiments were obtained from SIGMA and Wako Pure Chemical Industries. They were used without further purification. DNA oligos were synthesized by Eurofins Genomics, Tsukuba Oligo Service (Japan), and Hokkaido System Science (Japan).

PCR were performed with a Biometra TRIO48 thermalcycler. Real-time quantitative PCR (qPCR) were performed with a StepOne Real-Time PCR System, using THUNDERBIRD SYBR qPCR Mix (Toyobo, Japan). Unless otherwise stated, PrimeSTAR HS DNA polymerase (Takara) was used for PCR under the conditions recommended by the manufacturer, and DNA was purified by FavorPrep PCR Clean-Up Mini Kit (Favorgen). For primers, see the above-mentioned Table 2.

Gel images were taken with a Typhoon FLA9500 imager (GE healthcare). Unless otherwise stated, PAGE analysis of DNA and RNA were performed at 60 °C using gels containing 8 M urea, with 0.5 x TBE as running buffer. SDS-PAGE analysis of cDNA display molecules and peptide-linker complexes were performed at room temperature (r.t.) using Tris-HCl gels containing 8 M urea. Preparation of DNA samples for sequencing was performed according to the manufacturer's instruction.

### (2) Synthesis of cnvK-rG linker

cnvK-rG linker was synthesized from two modified oligonucleotides, puro-F-S2 fragment (5'-STCFCTC-(Spacer18)2-CCP-3') and biotin-rG-cnvK fragment (5'-BA-(rG)-AATTTCCAKGCCGCCCCCCG-(T-NH2)-CCT-3'), using the same protocol for cnvK-Pu linker described in our previous paper (Y. Mochizuki, T. Suzuki, K. Fujimoto and N. Nemoto, J. Biotechnol., 2015, 212, 174-180.), where S is 5'-thiol-Modifier C6, F is fluorescein-dT, P is puromycin CPG, and Spacer 18 is the spacer phosphoramidite 18, B is 5'-biotin-TEG, rG is guanine ribonucleotide, K is cnvK (Y. Yoshimura and K. Fujimoto, Org. Lett., 2008, 10, 3227-3230), and T-NH² is amino-modifier C6 dT (terminology is according to Tsukuba Oligo Service).

### (3) DNA construction

The construct coding BDA (Fig. 7A) was prepared according to a conventional method. The anti-GFP VHH construct (Fig. 7B) was prepared as follows. First, a gene coding anti-GFP VHH (LaG-16), which was chemically synthesized by GenScript, was amplified by PCR (25 cycles, 50 µL scale) with 1st Fw VHH and 1st Rv VHH as primers. Second PCR to attach 5'-UTR was then performed (25 cycles, 50 µL scale) with the above DNA as template and T7Ω and cnvK-NewYtag as primers. The product was purified by preparative PAGE (4%, denaturing conditions).

### (4) Preparation of cDNA display

Transcription of DNA was performed with T7 RiboMAX Large Scale RNA Production System (Promega) according to the manufacturer's instruction, and mRNA was purified by Agencourt RNA Clean XP (Beckman Coulter). mRNA (1 µM) was then hybridized to cnvK-rG linker (1 µM) at the 3'-terminal region in 25 mM Tris-HCl (pH 7.5) with 100 mM NaCl under the following annealing conditions: heating at 90 °C for 1 minutes followed by lowering the temperature to 70 °C at a rate of 0.4 °C/second, incubating for 1 minutes, then cooling to 25 °C at a rate of 0.08 °C/second.

The sample was irradiated with UV light at 365 nm using CL-1000 UV Crosslinker (UVP, Upland, USA) for 30 seconds. The obtained mRNA-linker complex was then in vitro translated with a rabbit reticulocyte lysate system (Promega, 6 pmol of mRNA-linker was added in 50 µL reaction volume) at 30 °C for 20 minutes. To synthesize an mRNA - linker - protein fusion (i.e. mRNA display or IVV), KCl and MgCl2were added to the mixture (final concentrations were 900 mM and 75 mM, respectively), and the mixture was incubated at 37 °C for 60 minutes.

After EDTA (final 70 mM) and equal volume of 2× SA binding buffer (20 mM Tris-HCl, pH 7.4, 2 mM EDTA, 2 M NaCl, 0.2% (v/v) Tween 20) was added, the mRNA display library was immobilized on streptavidin (SA)-coated magnetic beads (Dynabeads MyOne streptavidin C1 streptavidin magnetic beads, Invitrogen, 60 µL) at 25 °C for 30 minutes. The beads were washed three times with 1 × SA binding buffer (100 µL, 10 mM Tris-HCl, pH 7.4, 1 mM EDTA, 1 M NaCl, 0.1% (v/v) Tween 20).

The immobilized library was then reverse transcribed by ReverTra Ace reverse transcriptase (Toyobo, Japan, 200 U in 50 µL reaction volume) at 42 °C for 30 minutes. The beads were washed with His tag binding buffer (100 µL, 20 mM sodium phosphate buffer, 0.5 M NaCl, 5 mM imidazole, pH 7.4), and RNase T1 (Thermo Fischer Scientific) was added (250 U in 50 µL of His tag binding buffer) to the beads. The mixture was incubated for 30 minutes 37 °C to release the mRNA/cDNA - protein fusion molecules (i.e. cDNA display) from the beads.

Supernatant containing cDNA display molecules was collected, and purification using His6peptide tag was performed to remove contaminated cDNA-linker complex as follows. To His Mag Sepharose Ni beads (20 µL, GE healthcare) was added the above supernatant, and incubation was performed at 25 °C for 2 hours. The beads were washed with His tag wash buffer (100 µL, 20 mM sodium phosphate buffer, 0.5 M NaCl, 20 mM imidazole, pH 7.4), and incubated in His tag elution buffer (20 µL, 20 mM sodium phosphate buffer, 0.5 M NaCl, 250 mM imidazole, pH 7.4) at 25 °C for 15 minutes. The eluted fraction was used for immunoassay after appropriate dilution.

### (5) Confirmation of cDNA display formation of BDA by SDS-PAGE (Anti-GFP VHH, (Fig. 8))

The cDNA display coding BDA was prepared from 6 pmol of mRNA-linker complex as described above. Aliquots of mRNA-linker (lane 1), input (= IVV, lane 2) and supernatant (lane 3) of SA-beads immobilization, elution of RNase T1 treatment (lane 4, namely, crude cDNA display), supernatant of Ni beads (lane 5), and finally collected His tag elution (lane 6, namely, purified cDNA display) were taken and analyzed by SDS-PAGE (4% stacking-6% separating gel, 20 mA, 120 minutes) containing 8 M urea.

All samples corresponded to 0.5 pmol of molecules, assuming that every step proceeded with perfect yield. The gel was fluorescently visualized with FITC filter set (fluorescence of fluorescein attached to the cnvK-rG linker was visualized), and band intensity was calculated to estimate the cDNA display formation efficiency as well as absolute concentration of the final cDNA display solution. As to the last three samples, RNase H (Takara, 10 U) and 10× NE buffer 2 (1/10 volume, NEB) was added to the sample and the mixture was incubated at 37 °C for 30 minutes before loading to a gel, in order to digest RNA/cDNA duplex.

Quantification of band intensities of Fig. 8 indicated that total efficiency of cDNA display formation (from mRNA-linker to His tag elution) was about 7.7%.

Further purification with Ni-NTA beads (supernatant was analyzed in lane 5) yielded pure cDNA display molecules (lane 6). For lane 4-6, samples were loaded after RNase H treatment. Samples were analyzed by SDS-PAGE containing 8 M urea, and fluorescence of fluorescein attached to the cnvK-rG linker was visualized with gel imager. Quantification of band intensities indicated that total efficiency of cDNA display formation (from mRNA-linker to His tag elution) was about 7.7%.

### (6) Confirmation of cDNA display formation by SDS-PAGE (Anti-GFP VHH)

The anti-GFP VHH coding cDNA display was prepared from 6 pmol of mRNA-linker complex as described above. During cDNA display formation, the following aliquots were collected: the aliquots of mRNA-linker; input, namely, IVV; the supernatant of SA-beads immobilization; the eluate of RNase T1 treatment, namely, crude cDNA display; the supernatant of Ni beads; collected His tag elution, namely purified cDNA display; the final sample purified with Micro Bio-Spin 6 column, namely buffer exchange. Then, they were analyzed by SDS-PAGE (4% stacking-6% separating gel, 20 mA, 120 minutes) containing 8 M urea. All of the samples except the Bio-Spin 6 elution (1.7 pmol) corresponded to 0.5 pmol of molecules, assuming that every step proceeded with perfect yield. The gel was fluorescently visualized with FITC filter set, and band intensity was calculated to estimate the cDNA display formation efficiency. As to the last four samples, both of RNase H (Takara, 10 U) and 10× NE buffer 2 (1/10 volume, NEB) were added to the sample and the mixture was incubated at 37 °C for 30 minutes before loading to a gel, in order to digest RNA/cDNA duplex.

Lanes in Fig. 9 correspond to the following. Lane 1: mRNA-linker. Lane 2: IVV. Lane 3: supernatant of magnetic beads. Lane 4: crude cDNA display. Lane 5: supernatant of Ni beads. Lane 6: purified cDNA display molecules. Lane 7: the final sample purified with Micro Bio-Spin 6 column. Quantification of band intensities of Fig. 9 indicated that total efficiency of cDNA display formation (from mRNA-linker to Bio-Spin 6 elution) was about 0.3%.

### (Example 2)

### (1) Sensitivity assessment of cD-IPCR (Fig. 4A)

The BDA-coding cDNA display molecules were diluted every 10-fold (from 10³-10⁹ copies/ 2 µL) and 3.3-fold (10¹-10³ copies/2 µL) by ultrapure distilled water (UPDW), and 2 µL of these solutions were then used as a DNA template for qPCR. qPCR was performed with THUNDERBIRD SYBR qPCR Mix (Toyobo, Japan) using the StepOne Real-Time PCR System.

The PCR mixture contained 1 × THUNDERBIRD SYBR qPCR Mix, 300 nM concentration of each primer (forward primer: BDA_qPCR (+), reverse primer: BDA_qPCR (-)), 0.4 µL of 50× ROX reference dye, 2 µL of the above cDNA display dilutes and UPDW in a final volume of 20 µL. The step program for PCR was as follows: 95 °C for 1 minute, followed by 40 cycles of 95 °C for 15 sec and 62 °C for 30 second. The negative control that contained all the qPCR reagents except the DNA template was included to verify the quality of amplification, and the difference of Ct values between the samples and the control was calculated. As shown in Fig. 4A, ΔCt (difference of Ct values with respect to no template control) was linearly correlated with the number of cDNA display molecules in the range of 10³-10⁹ copies, and the standard deviations were very low. At lower concentrations, whereas linearity was not maintained, statistically significant ΔCt was observed for the sample containing as low as 100 molecules. Both of the low limit of detection (LOD) and the wide detection range (from 10² to 10⁹) are unique advantages of IPCR.

### (2) Immobilization of IgG to magnetic beads (Fig. 4B, Fig. 6, Fig. 10)

IgG from rabbit serum (Sigma, 1.5 nmol) in reaction buffer (0.1 M Na₂HPO₄, 0.1 M NaH₂PO₄, 0.3 M NaCl) was reacted with EZ-Link Sulfo-NHS-SS-Biotin (Thermo, 30 nmol) in reaction buffer at 25 °C for 30 minutes, and then the buffer was exchanged to 1 × SA binding buffer (10 mM Tris-HCl, pH 8.0, 1 mM EDTA, 1 M NaCl, 0.1% (v/v) Tween 20). Yield of collection was measured from absorbance of IgG.

Magnosphere MS300/Streptavidin (JSR, 20 µL, in the case of Fig. 6) or Dynabeads MyOne streptavidin C1 streptavidin magnetic beads (Invitrogen, 20 µL, in the case of Fig. 4B and Fig. 10) was washed with 1× SA binding buffer (100 µL), and reacted with the collected IgG (60 pmol) at 25 °C for 30 minutes, then washed out by 1 × SA binding buffer (100 µL, 3 times). Yields of immobilization reaction were estimated by subtracting absorbance of IgG in the flow through from that in the input, and were about 10% (Magnosphere MS300/Streptavidin) and 40% (Dynabeads MyOne streptavidin C1 streptavidin magnetic beads).

### (3) Comparison with blocking agents (Figs. 6 and 10A)

Each blocking agent was used for IgG-immobilized magnetic beads prepared as described above, and cDNA display of BDA was incubated with the beads. First, each blocking agent (5% (w/v) skim milk, 3% (w/v) BSA, 0.02% (w/v) yeast tRNA, 1% (w/v) gelatin from cold water fish, and 2.5%/0.01% (w/v) BSA/tRNA mixture) was dispersed in 1 × SA binding buffer (50 µL) and reacted with different concentration of IgG immobilized beads (10 µL) at 25 °C for 60 minutes.

Blocked beads were washed with 1 × SA binding buffer (100 µL, 3 times), and then reacted with cDNA display coding BDA that was diluted 5-fold with 1 × SA binding buffer (20 µL, i.e. the amount of cDNA display was not over than 0.08 pmol) at 25 °C for 60 minutes. The beads were washed 2 times with 1 × SA binding buffer (100 µL), and the beads suspension (2 µL) was directly used as qPCR template. The qPCR conditions were the same as above (see Sensitivity assessment of cD-IPCR).

Collected DNA was evaluated by qPCR. According to the results, we concluded that skim milk was the best blocking agent in combination with Dynabeads MyOne streptavidin C1 streptavidin magnetic beads.

### (Example 3)

### (1) Direct cD-IPCR detection of IgG (Fig. 4B and 10B)

In direct cD-IPCR, IgG immobilized Dynabeads MyOne streptavidin C1 streptavidin magnetic beads (prepared above) were mixed in 10-fold serial dilution with intact Dynabeads MyOne streptavidin C1 streptavidin magnetic beads (final concentrations of IgG were 0.16 to 1600 ng/mL). Beads were blocked with 5% (w/v) skim milk dispersed in 1×SA binding buffer (50 µL, 10 mM Tris-HCl, pH 7.4, 1mM EDTA, 1M NaCl, 0.1% (v/v) Tween 20). Blocked beads were washed twice with 1×SA binding buffer (100 µL), and then reacted with cDNA display coding BDA that was diluted 5-fold with 1×SA binding buffer (20 µL, i.e. the amount of cDNA display was ~80 fmol/sample) at 25 °C for 60 minutes. After washing the beads twice with 1×SA binding buffer (100 µL), the bound cDNA display was eluted by SDS/DTT buffer (100 µL, 1% (v/v) SDS, 50 mM DTT, 50 mM Tris-HCl, 0.5 M NaCl, 1mM EDTA, 0.05% (v./v) Tween 20, pH 7.4) at 50 °C for 30 minutes. After DNA purification (elution from spin column was performed with 30 µL water), samples were used for qPCR as a template (8 µL). The qPCR conditions were the same as above (see Sensitivity assessment of cD-IPCR). The difference of Ct values between the samples and the no antigen negative control (0 ng/mL IgG) was calculated.

The results of detection are shown in Fig. 4B and 10B. It was indicated that direct cD-IPCR could detect IgG from 1.6 µg/mL to 1.6 ng/mL.

### (2) Sandwich cD-IPCR detection of GFP in buffer (Fig. 4B)

The polystyrene microtiter plate (MICROLON, 96 Well Single-Break Strip Plate, PS, Greiner) was coated overnight at 4 °C with anti-GFP pAb (MBL, #598) diluted 1000-fold with PBS (10 mM sodium phosphate buffer containing 137 mM NaCl and 2.68 mM KCl, pH 7.4, 100 µL). After wash with PBS (200 µL), the plate was blocked with PBS containing 1% (w/v) skim milk by incubation at 25 °C for 2 hours, and then washed out by PBS (200 µL, 3 times). Each 10-fold serial dilutions of GFP in PBS from 100 µg/mL to 1 ng/mL were applied to the wells at 25 °C for 2 hours, and washed out by PBS (200 µL, 8 times).

cDNA display coding anti-GFP VHH, diluted by 65-fold with PBS-T (10 mM sodium phosphate buffer containing 137 mM NaCl and 2.68 mM KCl, 0.05% (v/v) Tween 20, pH 7.4, 100 µL), was then incubated at 25 °C for 2 hours. After wash by PBS-T (200 µL, 8 times), the bound cDNA display was eluted by glycine/HCl buffer (0.2 M, pH 2.2, 100 µL). The elute was neutralized with 1 M Tris-base solution, and cDNA display was purified by Favorprep kit for use as DNA template for qPCR. qPCR was performed with THUNDERBIRD SYBR qPCR Mix (Toyobo, Japan) using the StepOne Real-Time PCR System.

The PCR mixture contained 1 × THUNDERBIRD SYBR qPCR Mix, 300 nM concentration of each primer (forward primer: GFPVHHqPCRFw, reverse primer: GFPVHHqPCRRv), 0.4 µL of 50× ROX reference dye, 2 µL of purified elution cDNA display and UPDW in a final volume of 20 µL. The step program for PCR is as follows: 95 °C for 1 minutes, followed by 40 cycles of 95 °C for 15 sec and 66 °C for 30 second. The negative control that contained all the qPCR reagents except the DNA template is included to verify the quality of amplification.

Fig. 5 (E) shows detection of GFP in buffer with cD-IPCR method (** p < 0.01; *** p < 0.001, versus 0 ng/mL, Student t-test). Data are shown as mean ± S.D. (n = 3). The ΔCt value was gradually increased with the GFP concentration (from 10 to 10⁵ ng/mL), and 10 ng/mL was the lowest detected concentration.

### (3) Sandwich cD-IPCR detection of GFP in serum (Fig. 5F)

The procedures of the sandwich cD-IPCR detection of GFP in serum was similar to that of the "sandwich cD-IPCR detection of GFP in buffer" except that anti-GFP pAb was dissolved in commercially available healthy human serum (Kohjin Bio, Japan) instead of PBS, and used for the reaction.

Fig. 5 (F) shows the detection of GFP spiked in serum with cD-IPCR (*p < 0.05; ***p < 0.001, versus 0 ng/mL, Student t-test). Data are shown as mean ± S.D. (n = 3). The sensitivity of detection was essentially the same as in the buffer, which indicated that cD-IPCR could be applied for biological samples.

In contrast to ELISA, the signal amplification of cD-IPCR is exponential, resulting in very high sensitivity (~100 molecules/sample) and broad detection dynamic range (> 10⁷fold). Compared with traditional IPCR and PD-IPCR, this method has potential advantages in terms of quantitativity and reproducibility due to intrinsic one-to-one conjugation ratio of cDNA and its coding protein. Therefore, the method developed here has broad applicability and practical utility in immunoassays of a wide variety of antigens.

Also, like PD-IPCR, the newly developed method can be coupled with in vitro selection of optimized polypeptides from huge libraries. Of course, like other IPCR, the sensitivity of the assay highly depends on a number of factors including the affinity of the capture/detection antibodies, extent of non-specific binding of the DNA-antibody conjugates, and choice of primer pairs.

In conclusion, we established the novel PCR-based antigen detection method called cD-IPCR. cD-IPCR, which takes an advantage of the structural characteristics of cDNA display (a peptide-cDNA conjugate developed for in vitro evolution of polypeptides), proved to work in both direct-type and sandwich-type detection of target proteins, and detection of a target in serum was also possible. In contrast to ELISA, the signal amplification of cD-IPCR is exponential, resulting in high potential sensitivity (~100 molecules/sample) and a broad detection dynamic range (> 10⁷fold). After extensive optimization, this method may have advantages in terms of quantitativity and reproducibility compared with traditional IPCR and PD-IPCR, due to the intrinsic one-to-one conjugation ratio of cDNA and its coding protein.

### Industrial Applicability

The present invention is useful in medical, pharmaceutical and diagnosis field.

## Claims

1. Improved immuno-PCR method by using cDNA display comprising the steps of:
immobilizing a first antibody having a binding site to a solid phase:
contacting a sample fluid to the first antibody to bind a target molecule in said sample fluid;
contacting said target molecule to a cDNA display being composed of 1) a backbone nucleic acid strand in which a DNA sequence to be amplified is included and 2) a covalently-bound side chain strand having a polypeptide conjugation site to which a second antibody that binds to said target molecule has been conjugated; and
conducting polymerase chain reaction to amplify and detect said DNA quantitatively, wherein
said polypeptide conjugation site is composed of any one of compound selected from the group consisting of puromycin and derivative thereof,
said backbone nucleic acid strand comprises a double strand of mRNA and cDNA,
said backbone strand comprises the cDNA sequence of the second antibody which binds to the target molecule, and
the second antibody is a single domain antibody, that binds to a different epitope of the target molecule from the first antibody.

2. The improved immuno-PCR method by using cDNA display according to the claim 1, wherein the first antibody is bound to the solid phase directly.

3. The improved immuno-PCR method by using cDNA according to the claim 1, wherein the first antibody is bound to the solid phase via biotin-streptavidin interaction.

4. The improved immuno-PCR method by using cDNA according to any one of the claims 1 to 3, wherein the first antibody is anyone selected from the group consisting of IgG, a fragment thereof, and a single domain antibody that binds to epitope of the target molecule.

5. The improved immuno-PCR method by using cDNA according to any one of the claims 1 to 4, wherein said PCR is quantitative PCR.

## Patentansprüche

1. Verbessertes Immun-PCR-Verfahren unter Verwendung einer cDNA-Darstellung, umfassend die Schritte:
Immobilisieren eines ersten Antikörpers mit einer Bindungsstelle an einer festen Phase:
Inkontaktbringen eines Probenfluids mit dem ersten Antikörper, um ein Zielmolekül in dem Probenfluid zu binden;
Inkontaktbringen des Zielmoleküls mit einem cDNA-Display, das aus 1) einem Rückgrat-Nukleinsäurestrang, der eine zu amplifizierende DNA-Sequenz umfasst, und 2) einem kovalent gebundenen Seitenkettenstrang mit einer Polypeptidkonjugationsstelle, an die ein zweiter Antikörper, der an das Zielmolekül bindet, konjugiert wurde, besteht; und
Durchführen einer Polymerasekettenreaktion, um die DNA quantitativ zu amplifizieren und nachzuweisen, wobei
die Polypeptidkonjugationsstelle aus einer Verbindung besteht, die aus der Gruppe ausgewählt ist, bestehend aus Puromycin und Derivaten davon,
der Rückgrat-Nukleinsäurestrang einen Doppelstrang aus mRNA und cDNA umfasst,
der Rückgratstrang die cDNA-Sequenz des zweiten Antikörpers umfasst, der an das Zielmolekül bindet, und
der zweite Antikörper ein Einzel-Domänen-Antikörper ist, der an ein anderes Epitop des Zielmoleküls bindet als der erste Antikörper.

2. Verbessertes Immuno-PCR-Verfahren unter Verwendung einer cDNA-Darstellung nach Anspruch 1, wobei der erste Antikörper direkt an die feste Phase gebunden ist.

3. Verbessertes Immuno-PCR-Verfahren unter Verwendung von cDNA nach Anspruch 1, wobei der erste Antikörper über eine Biotin-Streptavidin-Wechselwirkung an die feste Phase gebunden ist.

4. Verbessertes Immun-PCR-Verfahren unter Verwendung von cDNA gemäß einem der Ansprüche 1 bis 3, wobei der erste Antikörper ausgewählt ist aus der Gruppe, bestehend aus IgG, einem Fragment davon und einem Einzel-Domänen-Antikörper, der an ein Epitop des Zielmoleküls bindet.

5. Verbessertes Immun-PCR-Verfahren unter Verwendung von cDNA gemäß einem der Ansprüche 1 bis 4, wobei die PCR eine quantitative PCR ist.

## Revendications

1. Méthode d'immuno-PCR améliorée utilisant un affichage d'ADNc, comprenant les étapes suivantes :
immobiliser un premier anticorps ayant un site de liaison à une phase solide ;
contacter un échantillon de fluide avec le premier anticorps afin de lier une molécule cible dans ledit échantillon de fluide ;
contacter ladite molécule cible avec un affichage d'ADNc composé 1) d'un brin d'acide nucléique de squelette dans lequel est incluse une séquence d'ADN à amplifier et 2) d'un brin de chaîne latérale lié de manière covalente ayant un site de conjugaison polypeptidique auquel a été conjugué un deuxième anticorps qui se lie à ladite molécule cible ; et
réaliser une réaction en chaîne par polymérase pour amplifier et détecter quantitativement ledit ADN, dans laquelle
ledit site de conjugaison polypeptidique est composé de l'un des composés choisis dans le groupe constitué par la puromycine et ses dérivés,
ledit brin d'acide nucléique de squelette comprend un double brin d'ARNm et d'ADNc,
ledit brin de squelette comprend la séquence d'ADNc du deuxième anticorps qui se lie à la molécule cible, et
le deuxième anticorps est un anticorps à domaine unique qui se lie à un épitope de la molécule cible différent de celui du premier anticorps.

2. La méthode immuno-PCR améliorée utilisant l'affichage d'ADNc selon la revendication 1, dans laquelle le premier anticorps est lié directement à la phase solide.

3. La méthode immuno-PCR améliorée utilisant l'ADNc selon la revendication 1, dans laquelle le premier anticorps est lié à la phase solide par interaction biotine-streptavidine.

4. La méthode immuno-PCR améliorée utilisant l'ADNc selon l'une des revendications 1 à 3, dans laquelle le premier anticorps est choisi parmi le groupe constitué par l'IgG, un fragment de celle-ci et un anticorps à domaine unique qui se lie à l'épitope de la molécule cible.

5. Procédé immuno-PCR amélioré utilisant l'ADNc selon l'une des revendications 1 à 4, dans lequel ladite PCR est une PCR quantitative.
